# EUROPEAN PATENT SPECIFICATION

(11) **EP 4 108 323 B1**
(45) Date of publication and mention of the grant of the patent: **15.10.2025**
(21) Application number: 21756542.3
(22) Date of filing: 16.02.2021
(51) Int. Cl.: A61Q 1/00, A61Q 19/00, A61K 9/107, A61K 8/06, A61K 8/60, A61K 47/26, A61K 47/22

(54) **STABILIZER FOR EMULSION COMPOSITION**
STABILISATOR FÜR EMULSIONSZUSAMMENSETZUNG
AGENT STABILISANT DE COMPOSITION D'ÉMULSION

(30) Priority: 17.02.2020 JP 2020024772
(43) Date of publication of application: 28.12.2022
(73) Proprietor: Nagase Viita Co., Ltd., Okayama-shi, Okayama 702-8006 (JP)
(72) Inventor: HANAOKA, Miho, Okayama-shi, Okayama 702-8006 (JP); OTAO, Masahiro, Okayama-shi, Okayama 702-8006 (JP); MIYAKE, Masaki, Okayama-shi, Okayama 702-8006 (JP); MITSUZUMI, Hitoshi, Okayama-shi, Okayama 702-8006 (JP)
(74) Representative: Page White Farrer
(86) International application number: PCT/JP2021/005757
(87) International publication number: WO 2021/166916

(56) References cited:
- JP-A- 2000 026 493
- JP-A- 2019 141 825
- JP-A- 2019 141 825
- JP-A- H0 413 691
- KR-A- 20180 026 989
- KR-A- 20180 026 989
- US-A1- 2009 239 958
- ZIJUN LUO ET AL: "Particle-Stabilizing Effects of Flavonoids at the Oil-Water Interface", JOURNAL OF AGRICULTURAL AND FOOD CHEMISTRY, vol. 59, no. 6, 23 March 2011 (2011-03-23), US, pages 2636 - 2645, XP055510342, ISSN: 0021-8561, DOI: 10.1021/jf1041855

## Description

### Technical Field

The present invention relates to a stabilizer for an emulsion composition. In more detail, the present invention relates to a stabilizer for an emulsion composition emulsified with a non-ionic surfactant, comprising a glycosyl naringenin as an active ingredient, as well as a stabilization method.

### Background Art

An emulsion composition (emulsion) generally means a dispersed solution system in which both dispersed phase and dispersion medium are liquids, and usually refers to a water-in-oil type or oil-in-water type dispersion solution, which are composed of an oil component and an aqueous component. Surfactant are generally used in order to emulsify the mixture of an oil component and an aqueous component. Use of a large amount of surfactant usually results in a long-term stabilization of the emulsified state of the emulsion composition.

Emulsion compositions are used in many fields including food, cosmetics, quasi-drugs, pharmaceuticals, etc. Particularly for emulsion compositions provided in the form of cosmetics, quasi-drugs, pharmaceuticals, etc. which are directly applied to the skin of human or animals, the high surfactant content of the emulsion composition disadvantageously gives rise to problems in physical properties, such as impaired feeling of use, as well as problems in safety, such as skin irritation. From this background, the reduction of the surfactant content of the emulsion composition is intensely desired.

For example, Patent Literature 1 discloses the surfactant-free method for improving the emulsifying activity and the feeling of use of an emulsion composition by adding alkyl-modified carboxy polymers and saccharides to the emulsion composition. Patent Literature 2 discloses the surfactant-free method for improving the stability and the feeling of use of an emulsion composition by controlling viscosity of the emulsion composition by addition of water-soluble synthetic polymers such as acrylic acid type polymer to the emulsion composition. Patent Literature 3 discloses the method for improving the emulsifying activity of an emulsion composition by including an alkyl esterified flavonoid glycoside compound in the emulsion composition. Patent Literature 4 discloses the surfactant-free method for improving the emulsifying activity and the feeling of use of an emulsion composition by including a polyphenol glycoside in the emulsion composition.

Unfortunately, addition of alkyl-modified carboxy polymers and saccharides did not provide satisfactory high emulsion stability, although the skin irritation problem was solved. On the other hand, addition of acrylic acid type polymers did not provide satisfactory good feeling of use, although satisfactory high emulsion stability was achieved. An alkyl-esterified flavonoid glycoside compound itself functioned as a surfactant and did not result in satisfactory good feeling of use. Furthermore, a polyphenol glycoside in the absence of surfactant did not provide satisfactory high emulsion stability. A further method for stabilizing an emulsion composition is intensely desired.

### Citation List

### Patent Literature

[Patent Literature 1] Japanese Patent Application Publication No. H8-217624
[Patent Literature 2] Japanese Patent Application Publication No. 2008-106043
[Patent Literature 3] Japanese Patent Application Publication No. 2019-141825
[Patent Literature 4] Japanese Patent Application Publication No. 2012-82183
[Patent Literature 5] Korean Patent Application Publication No. KR20180026989A discloses a method for producing a nanoemulsion with enhanced stability containing narirutin.
[Patent Literature 6] Japanese Patent Application Publication No. JP2019141825A relates to an emulsified composition having an emulsifying function, and in particular, to an emulsified composition having a high emulsifying function derived from nature and its use.
[Patent Literature 7] United States Patent Application Publication No. US2009239958A1 relates to a polyglycerol alkyl ether nonionic surfactants that exhibit superior emulsifying activities on a variety of oils and solvents; and to nonionic surfactant compositions containing the polyglycerol alkyl ether nonionic surfactants.

### Non-Patent Literature

Zijun Luo et al.: "Particle-Stabilizing Effects of Flavonoids at the Oil-Water Interface", JOURNAL OF AGRICULTURAL AND FOOD CHEMISTRY, vol. 59, no. 6, 23 March 2011 (2011-03-23), pages 2636-2645, XP055510342, US ISSN: 0021-8561, DOI: 10.1021/jf1041855 discloses a study showing that some common food flavonoids can act as excellent stabilizers of oil-in-water emulsions through their adsorption as water-insoluble particles to the surface of the oil droplets, i.e. Pickering emulsions are formed.

### Summary of Invention

### Technical Problem

The present invention was made in view of the prior art described above. The technical problem of the present invention is to provide a stabilizer for and a method for stabilizing an emulsion composition emulsified with a non-ionic surfactant.

### Solution to Problem

The present inventors accumulated research effort with the aim of solving the above technical problem and surprisingly found that the emulsified state of an emulsion composition can be maintained for a long duration when a glycosyl naringenin is mixed with and/or comprised in an emulsion composition emulsified with a non-ionic surfactant. The present invention was accomplished accordingly.

An embodiment of the present invention solves the above problem by providing a stabilizer for an emulsion composition emulsified with a non-ionic surfactant, the stabilizer comprising a glycosyl naringenin as an active ingredient. Another embodiment of the present invention solves the above problem by providing a method for stabilizing an emulsion composition emulsified with a non-ionic surfactant, the method comprising a step of having a stabilizer that comprises a glycosyl naringenin as an active ingredient mixed and/or contained in a starting material of the emulsion composition, an intermediate product of the emulsion composition, and/or the emulsion composition manufactured.

### Effects of Invention

In accordance with the present invention, the emulsion composition with high stability over time can be provided. Since the surfactant content of the emulsion composition can be reduced, higher formulation flexibility can be achieved as well as higher safety with a reduced risk of causing skin irritation, etc.

The stabilizer in accordance with the present invention can be applied to the skin of humans and animals on a daily basis, continuously, easily and safely without feeling uncomfortable. The stabilizer in accordance with the present invention can be industrially produced at low cost. Such a stabilizer of the present invention may be a useful stabilizer imparting stability to various formulations.

### Description of Embodiments

The present invention relates to a stabilizer for an emulsion composition emulsified with a non-ionic surfactant, comprising a glycosyl naringenin as an active ingredient, as well as a stabilization method.

The term "glycosyl naringenin" as referred to in this description refers to a type of flavanone and is a generic term of glycosides comprising "naringenin" having a structure of below chemical formula 1 as an aglycone. Representative examples of compounds included within the scope of "glycosyl naringenin" are "naringin" (below chemical formula 2), which has a structure composed of a naringenin and a neohesperidose (α-rhamnosyl (1→2)glucose) conjugated to the C(7) hydroxyl group of the naringenin by β-glycosidic linkage, and "3"-α-glucosyl naringin"(below chemical formula 3), which has a structure composed of a naringin and a glucose conjugated to the C(3) hydroxyl group of the glucose residue of the neohesperidose of the naringin (the C(3") hydroxyl group of the naringin) by α-glycosidic linkage.

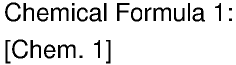

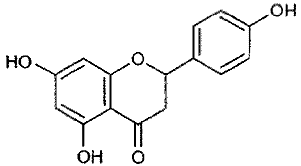

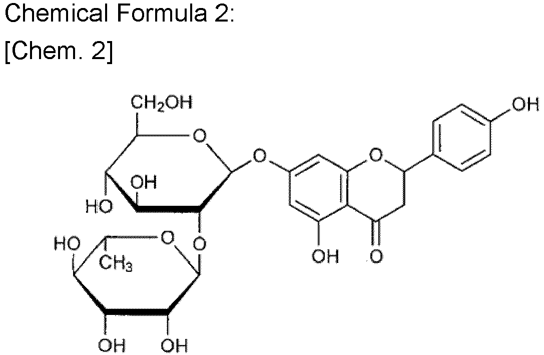

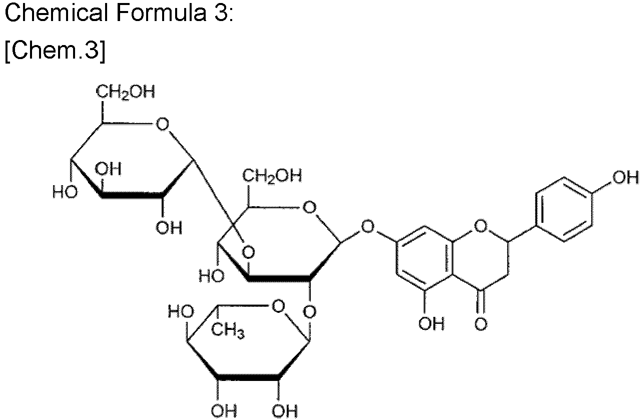

Moreover, within the scope of "glycosyl naringenin" as referred to in this description, "4'-α-glucosyl naringin"(below chemical formula 4), which has a structure composed of a naringin and a glucose conjugated to the C(4') hydroxyl group of the naringin by α-glycosidic linkage; "3"-α-,4'-α-diglucosyl naringin"(below chemical formula 5), which has a structure composed of a naringin and two glucoses, one conjugated to the C(3") hydroxyl group and another conjugated to the C(4') hydroxyl group of the naringin by α-glycosidic linkage; "prunin" (below chemical formula 6), which has a structure composed of a naringenin and a glucose conjugated to the C(7) hydroxyl group of the naringenin by β-glycosidic linkage; and further "narirutin" (below chemical formula 7), which has a structure composed of a naringenin and a rutinose (α-rhamnosyl (1→6) glucose) conjugated to the C(7) hydroxyl group of the naringenin by β-glycosidic linkage, are included.

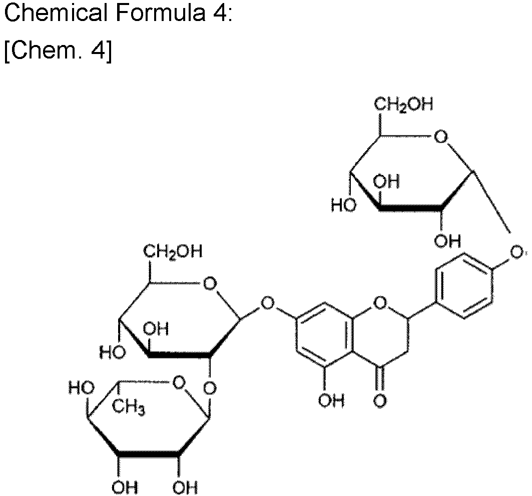

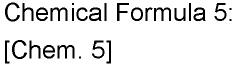

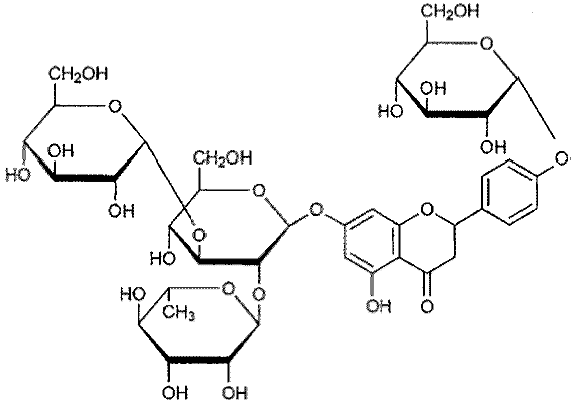

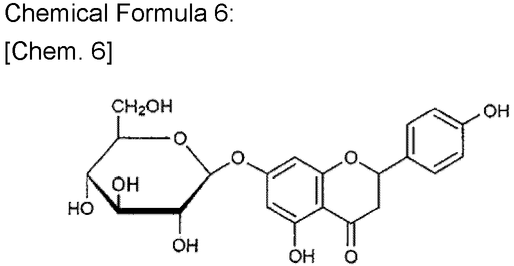

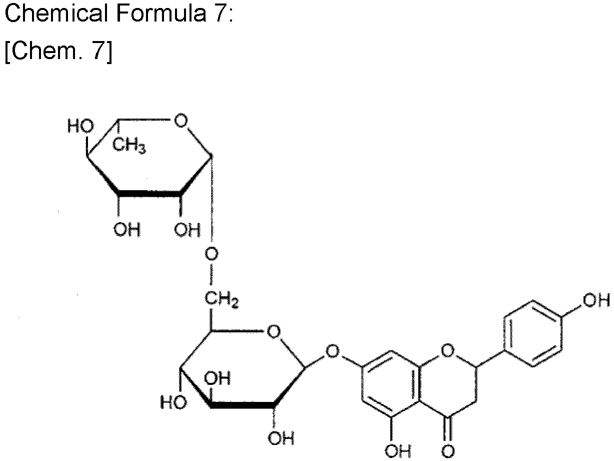

Furthermore, within the scope of "glycosyl naringenin" as referred to in this description, further glycosylated compounds of above described prunin, naringin, 3"-α-glucosyl naringin, narirutin, 4'-α-glucosyl naringin, and 3"-α-,4'-α-diglucosyl naringin, are also included. Examples of such glycosylated compounds include but not limited to α-maltosyl naringin, α-maltotriosyl naringin, α-maltotetraosyl naringin, α-maltopentaosyl naringin, α-glucosyl prunin, α-maltosyl prunin, α-maltotriosyl prunin, α-maltotetraosylprunin, α-maltopentaosyl prunin, α-glucosyl narirutin, α-maltosyl narirutin, α-maltotriosyl narirutin, α-maltotetraosylnarirutin, and α-maltopentaosyl narirutin.

The glycosyl naringenins may be prepared by enzymatic methods. If necessary, fermentation methods and chemical synthesis methods may be also used in preparing the glycosyl naringenins. From the viewpoint of economic efficiency, enzymatic methods using a glycosyltransferase may be advantageous for producing the glycosyl naringenins. For example, as a glycosyl naringenin, a series of 3"-α-glycosyl naringins having a glycosyl group with a glucose polymerization degree of 1 to 5 can be usually obtained at high yield, by allowing a glycosyltransferase, such as α-glucosidase, cyclomaltodextrin glucanotransferase, and α-amylase to act on naringin in the presence of an α-glucosyl saccharide compound, such as partial starch hydrolyzates and maltooligosaccharides as disclosed in Japanese Patent Application Publication No. H04-13691, Japanese Patent Application Publication No. 2007-284393, etc. Furthermore, 3"-α-glucosyl naringin may be advantageously prepared by allowing a glucoamylase to act on the series of 3"-α-glycosyl naringins.

The glucose polymerization degree of a glycosyl group of a glycosyl naringenin can be appropriately reduced by allowing a glucoamylase to act on the glycosyl naringenin. On the other hand, the glucose polymerization degree of a glycosyl group of a glycosyl naringenin can be advantageously increased by allowing a glycosyltransferase, such as cyclomaltodextrin glucanotransferase, to act on the glycosyl naringenin in the presence of a glycosyl donor, such as partial starch hydrolyzates. If necessary, modified glycosyl naringenins may be also prepared advantageously by further transferring a monosaccharide other than D-glucose, a disaccharide, an oligosaccharide, a polysaccharide, and so on, to a glycosyl naringenin by transglycosylation.

Prunin may be prepared by removing a rhamnose residue from a naringin by allowing a rhamnosidase to act on the naringin, as disclosed for example in Japanese Patent Application Publication No. 2007-284393. On the other hand, narirutin may be prepared, for example, by transferring a rhamnose to a prunin by α-1,6 transglycosylation. These glycosyl naringins may be also prepared using fermentation methods, chemical decomposition methods, and chemical synthesis methods, if necessary.

In the present invention, any glycosyl naringenin showing the stabilizing effect of the emulsion composition may be used and it may be of any origin, may be produced by any method, may be of any purity, etc., and may not necessarily be highly purified. As long as the desired effect and safety can be achieved, a glycosyl naringenin in the form of a crude enzymatic reaction solution, in the form of an unseparated composition comprising the glycosyl naringenin and other products inherent to its production method, in a partially purified form, and in a highly purified form may be also used. The purity of a glycosyl naringenin used in the stabilizer for an emulsion composition of the present invention may be usually 20 % by mass or more, preferably 40 % by mass or more, and more preferably 50 to 99.99 % by mass based on the solid content of the glycosyl naringenin. The highly purified glycosyl naringenin that is obtained through the crystallization process may be also used.

As described above, a glycosyl naringenin comprised in the stabilizer of the present invention, may be in the form of a composition, in other words a glycosyl naringenin mixture. Depending on its production process, this glycosyl naringenin mixture usually comprises as a main ingredient one or more kinds selected from (1) naringin and α-glycosyl naringin (e.g., α-glucosyl naringin, etc.), which are the compounds having a naringenin skeleton. The glycosyl naringenin mixture may further comprise (2) a flavonoid, such as diosmin and neoponcirin, and (3) a trace component, such as salts. These substances are considered to be derived from the raw material used in the production or produced as a by-product in the production process. The glycosyl naringenin mixture may comprise a naringenin, an aglycone of the glycosyl naringenin, to the extent that the desired effect of the present invention can be achieved. The stabilizer of the present invention may comprise a naringenin with enhanced dispersibility in water or in other solvents, to the extent that the desired effects of the present invention can be achieved. Such a naringenin with enhanced dispersibility in water or in other solvents may be the one prepared using a known physical method, such as particle size reduction, using a known chemical method, such as film-coating of particle and immobilization to microcarriers, or using other methods.

The stabilizer of the present invention may be the one comprising only one kind of a compound that is within the scope of the glycosyl naringenin. However, from the viewpoint of synergistically enhancing the effect of stabilizing an emulsion composition shown by the glycosyl naringenin, the stabilizer of the present invention may preferably comprise two or more kinds of compounds that are within the scope of the glycosyl naringenin. From the viewpoint of achieving high stabilization effect and water solubility, the stabilizer of the present invention may preferably comprise α-glycosyl naringin as the glycosyl naringenin, more preferably comprise α-glucosyl naringin as α-glycosyl naringin. Further preferably, the stabilizer of the present invention may comprise one or more selected from 3"-α-monoglucosyl naringin, 3"-α-,4'-α-diglucosylnaringin, and 4'-α-glucosyl naringin, as α-glucosyl naringin since the desired effect of the present invention (i.e., stabilization effect of an emulsion composition) can be more remarkably produced.

The stabilizer of the present invention may comprise the glycosyl naringenin in any suitable amount, which may be appropriately selected depending on the formulation form of the stabilizer or other ingredients contained in the stabilizer, provided that the desired effect of the present invention can be achieved. The glycosyl naringenin content of the stabilizer may be usually 50% by mass or more and 100% by mass or less, preferably 60% by mass or more and 100% by mass or less, more preferably 70% by mass or more and 100% by mass or less, more preferably 80% by mass or more and 100% by mass or less, and further preferably 85% by mass or more and 100% by mass or less based on the dry solid content of the stabilizer. The upper limit of the glycosyl naringenin content of the glycosyl naringenin to be contained in the stabilizer of the present invention may be usually 99% by mass or less based on the dry solid content, since the glycosyl naringenin having such a glycosyl naringenin content can be produced on an industrial scale in a relatively large amount, at relatively low cost, and relatively easily. From the viewpoint of further reducing cost, the upper limit of the glycosyl naringenin content of the glycosyl naringenin to be contained in the stabilizer may be as low as 80% by mass or less, or further as low as 60% by mass or less. However, when the glycosyl naringenin content of the glycosyl naringenin based on the dry solid content is too low, it becomes inevitable to use a larger amount of the glycosyl naringenin compared to when using the glycosyl naringenin with a high glycosyl naringenin content, and the operation becomes more complex and the handleability becomes poorer accordingly. From this viewpoint, the lower limit of the glycosyl naringenin content of the glycosyl naringenin may be usually 10% by mass or more, preferably 20% by mass or more, more preferably 30% by mass or more, more preferably 40% by mass or more, and further preferably 50% by mass or more based on the dry solid content of the glycosyl naringenin.

In a preferred embodiment, the stabilizer of the present invention may comprise an α-glycosyl naringenin as the glycosyl naringenin. In a further preferred embodiment, the stabilizer of the present invention may comprise an α-glucosyl naringin as the α-glycosyl naringenin. In this case, the α-glucosyl naringin content of the glycosyl naringenin may be usually 10% by mass or more and 100% by mass or less, preferably 20% by mass or more and 100% by mass or less, and more preferably 30% by mass or more and 100% by mass or less. The upper limit of the α-glucosyl naringin content of the glycosyl naringenin to be contained in the stabilizer of the present invention may be basically 100% by mass or less as described above, but, from the viewpoint of providing the stabilizer of the present invention at lower cost, it may be usually 99% by mass or less, preferably 95% by mass or less, and more preferably 90% by mass or less, since the glycosyl naringenins having such α-glucosyl naringin contents can be produced on an industrial scale in a relatively large amount, at relatively low cost, and relatively easily. On the other hand, from the same reason as described above for the lower limit of the glycosyl naringenin content of the glycosyl naringenin mixture, the lower limit of the α-glucosyl naringin content of the glycosyl naringenin may be usually 10% by mass or more, preferably 20% by mass or more, more preferably 30% by mass or more, more preferably 40% by mass or more, and further preferably 50% by mass or more based on the dry solid content of the glycosyl naringenin.

The stabilizer of the present invention may be added to and contained in any emulsion composition emulsified with a non-ionic surfactant. Such an emulsion composition may be an external dermal composition or a composition for oral ingestion. The stabilizer of the present invention may be added to, mixed with, and contained in these emulsion compositions, and function as a stabilizer for the emulsion compositions. Since the glycosyl naringenins used in the present invention may also have an effect of inhibiting elastase activity, anti-aging effect, anti-wrinkle effect (wrinkle prevention effect and wrinkle improvement effect), anti-sagging effect (sagging prevention effect and sagging improvement effect), slimming effect, blood circulation promoting effect, an effect of inhibiting lipase activity, radical scavenging effect, anti-inflammatory effect, whitening, brightening, or lightening effect, an effect of reducing lipid peroxide in the living body, effect of inhibiting decrease of ascorbic acid in the living body, and reactive oxygen species scavenging effect, in addition to the emulsion stabilization effect, the emulsion composition, such as the one in the form of an external dermal composition, comprising the stabilizer of the present invention may preferably comprise one or more kinds of the glycosyl naringenin in an amount sufficient as a whole for achieving the effects as exemplified above.

The glycosyl naringenin content of the emulsion composition, which the stabilizer of the present invention is added to, mixed with, and contained in, is usually from 0.001 to 20.0% by mass, preferably from 0.01 to 10.0% by mass, and more preferably from 0.05 to 5.0% by mass based on the total mass of the emulsion composition. The glycosyl naringenin content of less than 0.001% by mass based on the total mass of the emulsion composition may not be preferable, because the emulsion stabilization effect by the glycosyl naringenin may not be sufficiently exhibited. On the contrary, the glycosyl naringenin content of more than 20.0% by mass based on the total mass of the emulsion composition may not be preferable, because the emulsion stabilization effect by the glycosyl naringenin may not be sufficiently exhibited and the physical property, the pharmacological effect, etc. desired for the emulsion composition to which the stabilizer of the present invention is added may be impaired. From another aspect, the formulation ratio of the glycosyl naringenin relative to the non-ionic surfactant in the emulsion composition may be usually from 0.002 to 40.0 parts by mass, preferably from 0.02 to 20.0 parts by mass, and more preferably from 0.1 to 10 parts by mass of the glycosyl naringenin in total relative to 1 part by mass of the non-ionic surfactant in the emulsion composition. The formulation ratio of less than 0.002 parts by mass of the glycosyl naringenin relative to 1 part by mass of the non-ionic surfactant may not be preferable, because the emulsion stabilization effect by the glycosyl naringenin may not be sufficiently exhibited. On the contrary, the formulation ratio of more than 40.0 parts by mass of the glycosyl naringenin relative to 1 part by mass of the non-ionic surfactant may not be preferable, because the emulsion stabilization effect by the glycosyl naringenin may not be sufficiently exhibited and the physical property, the pharmacological effect, etc. desired for the emulsion composition to which the stabilizer of the present invention is added may be impaired.

The "non-ionic surfactant" as referred to in this description may be, for example, any of an ester type surfactant (polyol type surfactant), such as glycerin fatty acid ester, sorbitan fatty acid ester, and sucrose fatty acid ester; an ether type surfactant, such as polyoxyethylene alkyl ether, polyoxyethylene alkyl phenyl ether, and polyoxyethylene-polyoxypropylene glycol; an ester-ether type surfactant, such as polyethylene glycol fatty acid ester and polyoxyethylene sorbitan fatty acid esters; an alkanolamide type surfactant, such as fatty acid alkanolamide; and an alkyl-polyglucoside type surfactant. The stabilizer of the present invention may be suitably used in the emulsion composition comprising one or more of the non-ionic surfactants.

Although the mechanism underlying the stabilization of the emulsion composition emulsified with the non-ionic surfactant by the glycosyl naringenin comprised in the stabilizer of the present invention as an active ingredient is not fully elucidated, it is presumably because the glycosyl naringenin enhances the action of the non-ionic surfactant.

In accordance with the stabilizer of the present invention, not only an effect of enhancing the emulsion stability of the emulsion composition but also an effect of improving a variety of physical properties, which is inherent in the glycosyl naringenin comprised in the stabilizer as an active ingredient, as well as an effect of enhancing an action of the non-ionic surfactant are expected. Consequently, the stabilizer of the present invention may be added to and contained in emulsion compositions, for example, including not only a stabilizer, emulsification aid, and agent to improve physical property, but also a skin feeling improving agent, gloss agent, water retention agent, moisturizer, thickener, quality improving agent, excipient, adhesive, osmoregulation agent, flavor improving agent, anti-clouding agent, shelf-life extending agent, yield improving agent, curing regulator, flow regulating agent, viscoelasticity improving agent, adhesion improving agent, antioxidant, anti-browning agent, syneresis inhibitor, shaping agent, shape retention agent, refrigeration resistance imparting agent, freezing resistance imparting agent, ice crystal stabilizer, intestinal regulator, agent for improving feeling going down the throat, cleaning agent, detergent, toothpaste, softener, anti-static agent, disinfectant, solubilizer, dispersant, water repellent, blowing agent, foaming agent, anti-foaming agent, moisturizer, penetrant, glidant, surface treatment agent, spreader, lubricant, antirust agent, surface modifier, flocculant, solubilizer, anti-fogging agent, mold release agent, vulcanization accelerator, de-inking agent, dust inhibitor, oil spill treating agent, fire extinguishing agent, etc. The emulsion compositions comprising the stabilizer of the present invention may be advantageously used in a variety of compositions, such as food, recreational foods, drinks, and drugs, livestock feed, fishery feed, cosmetics, quasi-drugs, pharmaceuticals, industrial products, etc.

The stabilizer of the present invention may further comprise other conventional ingredients, depending on the form of a composition which the stabilizer is to be added to, mixed with, and contained in. In other words, when the stabilizer is to be added to and contained in an external dermal composition or a composition for oral ingestion, the stabilizer of the present invention may comprise one or more ingredients that are usually contained in such compositions to the extent that the desired effect of the present invention is not impaired.

The stabilizer of the present invention in any dosage form may be contained in the emulsion composition which is in the form of an external dermal composition, and the stabilizer of the present invention in a variety of dosage forms, for example, in an aqueous solution form, solubilized form, emulsion form, dispersion form, and solid dosage form, may be used. Meanwhile, the stabilizer of the present invention may be added to, mixed with, and contained in the external dermal composition in any form and the form of the composition is not bound by legal classification, such as cosmetics, quasi-drugs, and pharmaceuticals as stipulated in Japanese Act on Securing Quality, Efficacy and Safety of Products Including Pharmaceuticals and Medical Devices. The term "external dermal composition" as used in this description means an external dermal composition that is applied to the exodermis, such as the skin, the lip, and the scalp, and the oral cavity as cosmetics, quasi-drugs, or pharmaceuticals. Examples of such external dermal compositions include an ointment, cream, milky lotion, essence, toner, lotion, jelly, gel, pack, shampoo, rinse, hair treatment, mask, mascara, eyeliner, hair growth product, hair restoration product, lip balm, lipstick, lip gloss, foundation, blush, eyeshadow, soap, body wash, bathing agent, dentifrice, mouth freshener, mouth refreshing film, mouthwash, and medicated gargle.

The stabilizer of the present invention may be added to, mixed with, and contained in any emulsion composition emulsified with a non-ionic surfactant as an agent for stabilizing emulsion state of the composition. Examples of such emulsion compositions include but not limited to an external dermal agent for anti-wrinkling, external dermal agent for slimming, external dermal agent for promoting blood circulation, external dermal agent for inhibiting lipase activity, external dermal agent for anti-inflammation, external dermal agent for radical scavenging, external dermal agent for skin whitening, skin brightening or skin lightening, external dermal agent for reducing lipid peroxide in the living body, external dermal agent for preventing decrease of ascorbic acid in the living body, external dermal agent for anti-aging, external dermal agent for anti-dullness for the skin and the lip, external dermal agent for scalp care, external dermal agent for hair care, external dermal agent for anti-inflammation, makeup cosmetics, external dermal agent for massage, etc.

The stabilizer of the present invention may be added to, mixed with, and contained in any composition for oral ingestion that is emulsified with a non-ionic surfactant, and it is possible to obtain the similar effect as when the stabilizer is added to, mixed with, and contained in an external dermal composition that is emulsified with a non-ionic surfactant. The stabilizer of the present invention may be contained in a composition for oral ingestion in any dosage form, including, for example, an aqueous solution form, solubilized form, emulsion form, dispersion form, solid dosage form, and so on, and the desired effect of the present invention can be achieved. Moreover, the stabilizer of the present invention may be added to, mixed with, and contained in a composition for oral ingestion in any form, and the form of the composition for oral ingestion is not bound by classification, such as food and beverages, supplements, food for specified health uses, functional food, health food, dietary health supplement, food with health claims, dietary nutritional supplement, food with nutrient function claims, food with functional claims, food for special dietary uses, quasi-drugs, pharmaceuticals, etc. The term "composition for oral ingestion" as referred to in this description means a composition that is orally ingested as food, beverages, etc., and including the ones used for the purpose of skin whitening, brightening, or lightening, skin beautifying, slimming, promoting blood circulation, anti-inflammation, anti-wrinkling, anti-sagging, reducing lipid peroxide in the living body, radical scavenging, scavenging reactive oxygen species, preventing decrease of ascorbic acid in the living body, reducing body fat, reducing visceral fat, fatigue recovery, and reducing stress, etc. When the stabilizer of the present invention is added to, mixed with, and contained in the emulsion composition for oral ingestion, the glycosyl naringenin content of the emulsion composition may be from 0.001 to 20.0% by mass, preferably from 0.01 to 10.0% by mass, and more preferably from 0.05 to 5.0% by mass. When the glycosyl naringenin is used for the fortification purpose of achieving, for example, the above-described physiological effects, the stabilizer of the present invention itself may be advantageously used as the composition for oral ingestion. When orally ingesting the composition for oral ingestion comprising the stabilizer of the present invention, the amount of daily intake may be any amount, as long as the emulsion stabilization effect and other physiological effects can be achieved. For example, when a person with a body weight of 50 kg intakes the composition, the amount of daily intake may be 0.005 to 10 g per day, preferably 0.01 to 5 g per day, and more preferably 0.02 to 2.5 g per day in terms of the amount of the glycosyl naringenin.

The present invention is explained in further detail with reference to the following experiments.

### <Experiment 1: Effect of Glycosyl Naringenins and Type of Surfactants on Emulsion Stability of Emulsion Compositions>

Emulsion compositions comprising 3"-α-glucosyl naringin as glycosyl naringenin and a various types of surfactants were prepared and the emulsion stability of the prepared emulsion compositions was evaluated.

### <Experiment 1-1: Preparation of 3"-α-Glucosyl Naringin Composition>

Glycosyl naringenin was prepared in accordance with the method described in Example 1 of Japanese Patent Application Publication No. 2002-199896. In short, 50 parts by mass of naringin and 200 parts by mass of dextrin (Dextrose Equivalent (DE) 8) were dissolved in 500 parts by mass of water under heating, and the pH of the resultant solution was adjusted to 7.0 by addition of 2N sodium hydroxide solution. After the adjustment of pH, cyclodextrin glucanotransferase (Hayashibara Co., Ltd., Japan) from *Bacillus stearothermophilus* in an amount of 15 units/g-dextrin was added to the solution and the solution was subjected to a reaction for 48 hours at 68°C. After the reaction, the solution was heated to inactivate the enzyme and filtered to obtain a reaction solution comprising α-glycosyl naringin. Then, the pH of this reaction solution comprising α-glycosyl naringin was adjusted to 4.5, and glucoamylase (Product Name: Glucozyme; Amano Enzyme Inc., Japan) in an amount of 100 units/g-dextrin was added to the reaction solution, and the reaction solution was subjected to a reaction for 24 hours at 55°C, to produce α-glucosyl naringin from α-glycosyl naringin. Then, α-glucosidase (Product Name: Transglucosidase L "Amano", Amano Enzyme Inc., Japan) in an amount of 1 mL/g-α-glycosyl naringin was further added to the reaction solution and the reaction solution was subjected to a reaction for 24 hours at 55°C, to produce 3"-α-glucosyl naringin and naringin by decomposition of 3"-α-,4'-α-diglucosylnaringin and 4'-α-glucosyl naringin, respectively. After the reaction, the resultant reaction solution was heated to inactivate the enzyme and then filtered. The resulting filtrate was fed to a column packed with a porous synthetic adsorbent at a space velocity (SV) of 2, and α-glucosyl naringin and naringin were adsorbed to the column. The column was washed with water to wash out glucose, salts, etc., which did not adsorb to the column. Thereafter, the column was fed with an aqueous ethanol solution and the concentration of ethanol in the aqueous ethanol solution was increased in a stepwise manner, to collect an eluent comprising 3"-α-glucosyl naringin and naringin. The obtained eluent was concentrated *in vacuo* and pulverized, to obtain an α-glucosyl naringin composition. The HPLC analysis revealed that the α-glucosyl naringin composition was composed of 70% of glucosyl naringin (3"-α-glucosyl naringin) and 30% of naringin in the molar ratio (equivalent to the molar ratio of naringenin aglycone). The HPLC analysis was performed under the following condition.

### <Condition for HPLC Analysis>

Column: "CAPCELL PAK C18 UG 120" (Shiseido Company, Limited, Japan)
Eluent: Water/Acetonitrile/Acetic acid = 80/20/0.01 (v/v)
Detection: UV 280 nm
Temperature: 40°C
Flow rate: 0.8 mL/min

### <Experiment 1-2: Preparation of Test Emulsion Composition and Emulsion Stability Test>

After 14.4 g of the α-glucosyl naringin composition obtained in Experiment 1-1 was put in a polypropylene container, 985.6 g of ultrapure water was further added to the container. The α-glucosyl naringin composition was dissolved, to obtain a solution containing 1.44% by mass of the α-glucosyl naringin composition. The obtained solution was stored in a water bath at 80°C until use. Next, 10.5 g of liquid paraffin (Product Name: MORESCO WHITE P-70; MORESCO Corporation, Japan) as an oil component and 175 mg of a surfactant were taken in another polypropylene container, and were dissolved and mixed on a water bath at 80°C, to obtain an oil-surfactant mixture. The oil-surfactant mixture was stored in a water bath at 80°C until use. Then, 24.3 g of the above solution containing 1.44% by mass of the α-glucosyl naringin composition was gradually added to the oil-surfactant mixture on a water bath at 80°C, while homogenizing the oil-surfactant mixture at 4,000 rpm using a POLYTRON^{®} homogenizer (PT2500E, shaft diameter: ø 12 mm) (Kinematica AG, Switzerland). After that, the resultant mixture was further homogenized at 4,000 rpm for 3 mins on a water bath at 80°C, and then the resultant mixture was taken out of a water bath and allowed to cool under room temperature. During this cooling process, the mixture was kept homogenized at 4,000 rpm. The homogenization was terminated when the temperature of the mixture decreased to below 40°C, and thus a test emulsion composition was obtained. Control emulsion compositions were prepared in accordance with the method described above except that ultrapure water was used instead of the solution containing 1.44% by mass of the α-glucosyl naringin composition. Compositions of the test emulsion composition and the control emulsion compositions were shown in Table 1. Eleven types of surfactants used in this experiment were listed in Table 2.

Test emulsion compositions were incubated for 30 mins at room temperature after preparation, moved to a temperature of 30°C, and further incubated for 1 hour. Then, the emulsion stability of test emulsion compositions was visually evaluated. The emulsion stability was evaluated based on the degree of separation of the oil layer and the aqueous layer observed in the compositions incubated for 1 hour at 30°C. The emulsion stability of each test emulsion composition was judged "X" when the degree of separation was comparable to the corresponding control emulsion composition. Meanwhile, the emulsion stability was judged "O" when less separation and better emulsion state were observed compared to the corresponding control composition. The obtained result was shown in Table 2.

**[Table 1]**

| | Control Compositions | Test Emulsion Compositions |
|---|---|---|
| Liquid Paraffin | 30.0% by mass | 30.0% by mass |
| Surfactant | 0.5% by mass | 0.5% by mass |
| α-Glucosyl Naringin Composition obtained in Experiment 1-1 | - | 1.0% by mass |
| Ultrapure Water | Remaining Amount | Remaining Amount |
| Total | 100% by mass | 100% by mass |

**[Table 2]**

| Surfactant No. | Compund Name | Product Name | Manufacturer | Sufactant Type | | Result |
|---|---|---|---|---|---|---|
| 1 | Sodium stearate | NONSOUL SN-1 | NOF Corporation | Ionic (Anionic) | Aliphatic monocarboxylic acid salt | x |
| 2 | Sodium lauryl sulfate | ALSCOAP LN-90PW | TOHO Chemical Industry Co.,Ltd. | | Alkyl sulfonic acid salt | x |
| 3 | Potassium cocoyl glycinate | AMILITE GCK-11F | AJINOMOTO HEALTHY SUPPLY CO., INC. | | N-acyl amino acid salt | x |
| 4 | Potassium N-myristoyl-L-glutamate | AMISOFT MK-11F | AJINOMOTO HEALTHY SUPPLY CO., INC. | | N-acyl amino acid salt | x |
| 5 | Distearyldimethylammonium chloride | CATION DSV | Sanyo Chemical Industries, Ltd. | Ionic (Cationic) | Dialkyldimethylammonium chloride | x |
| 6 | Lauryl dimethylaminoacetic acid betaine | AMOGEN S-H | DKS Co. Ltd. | Ionic (Amphoteric) | Alkyl betaine | x |
| 7 | Cocamidopropyl betaine | TEGOBETAIN 70k | Evonik Japan Co., Ltd. | | Fatty acid amido propyl betaine | x |
| 8 | Sodium laurylaminodiacetate | NISSANANON LA | NOF Corporation | | Carboxymethyl amine | x |
| 9 | Polyoxyethylene behenyl ether 10EO | NIKKOL BB-10 | Nikko Chemicals Co., Ltd. | Non-ionic | Polyoxyethylene alkyl ether | ○ |
| 10 | Sorbitan monostearate | RHECDOL AS-10V | Kao Corporation | | Sorbitan fatty acid ester | ○ |
| 11 | Palm kernel fatty acid diethanolamide | AMISOL KD-1 | Kawaken Fine Chemicals Co.,Ltd. | | Fatty acid alkanol amide | ○ |

As a result of the stability test of the emulsion compositions prepared with different combinations of 11 kinds of surfactants and the glycosyl naringenin, it was surprisingly revealed that only the emulsion compositions prepared with non-ionic surfactants and the glycosyl naringenin showed the remarkably higher emulsion stability compared to the corresponding control emulsion compositions, which were prepared in the same manner except that the solution containing the α-glucosyl naringin composition was not included. In contrast, the separation was observed for all emulsion compositions prepared with ionic surfactants of any type, including anionic, cationic and amphoteric surfactants, revealing that the emulsion stability of these emulsion compositions was low and comparable to that of each corresponding control emulsion composition, which was prepared in the same manner except that the solution containing the α-glucosyl naringin composition was not included.

It was revealed that the α-glucosyl naringin composition selectively enhances the stability of an emulsion composition emulsified with a non-ionic surfactant. This result indicates that the stabilizer of the present invention is useful as a stabilizer for an emulsion composition emulsified with a non-ionic surfactant.

### <Experiment 1-3: Preparation of Test Emulsion Composition and Emulsion Stability Test - part 2>

The further emulsion stability test was performed with a focus on non-ionic surfactants. As shown in Table 3, test emulsion compositions were prepared in accordance with the method described in Experiment 1-2 except that glyceryl oleate (Product Name "NIKKOL MGO"; Nikko Chemicals Co., Ltd., Japan) or glyceryl isostearate (Product Name "NIKKOL MGIS"; Nikko Chemicals Co., Ltd., Japan) were used as a non-ionic surfactant in preparing each test emulsion composition.

Test emulsion compositions were incubated for 30 mins at room temperature after preparation, moved to a temperature of 30°C, and further incubated at the temperature for 3 days. Then, the emulsion stability of test emulsion compositions was visually evaluated. The emulsion stability was evaluated based on the degree of separation of the oil layer and the aqueous layer observed in the compositions incubated for 3 days at 30°C. The emulsion stability of each test emulsion composition was judged "X" when the degree of separation was comparable to the corresponding control emulsion composition. Meanwhile, the emulsion stability was judged "O" when less separation and better emulsion state were observed compared to corresponding control composition. The obtained result was shown in Table 3.

**[Table 3]**

| Surfactant No. | Compund Name | Product Name | Manufacturer | Sufactant Type | | Result |
|---|---|---|---|---|---|---|
| 12 | Glyceryl oleate | NIKKOL MGO | Nikko Chemicals Co., Ltd. | Non-ionic | Glycerin fatty acid ester | ○ |
| 13 | Glyceryl isostearate | NIKKOL MGIS | Nikko Chemicals Co., Ltd. | | | ○ |

As shown in Table 3, the stability test of the emulsion compositions prepared with the α-glucosyl naringin composition and either glyceryl oleate (No. 12) or glyceryl isostearate (No. 13), both of which are a non-ionic surfactant categorized as glycerin fatty acid ester, revealed that these emulsion compositions showed the remarkably higher emulsion stability compared to the corresponding control emulsion compositions, which were prepared in the same manner except that the solution containing the α-glucosyl naringin composition was not included. This result was consistent with the result of Experiment 1-2.

This result indicates that the α-glucosyl naringin composition enhances the stability of an emulsion composition emulsified with an ester type non-ionic surfactant, not only when sorbitan fatty acid ester (sorbitan monostearate (No.10)) shown in the result of Experiment 1-2 (Table 2) but also when glycerin fatty acid ester is used as the ester type non-ionic surfactant.

### <Experiment 2-1: Effect of Mass Ratio of Glycosyl Naringenin and Surfactants on Stability of Emulsion Compositions>

Emulsion compositions with different mass ratio of a glycosyl naringenin and a non-ionic surfactant were prepared using the α-glucosyl naringin composition prepared in Experiment 1-1 as the glycosyl naringenin and sorbitan monostearate as the non-ionic surfactant, and the emulsion stability of the emulsion compositions was evaluated.

### <Preparation of Test Emulsion Compositions and Emulsion Stability Test>

Test emulsion compositions 1 to 12 were prepared in accordance with the method described in Experiment 1 except that the final concentration of the α-glucosyl naringin composition prepared in Experiment 1-1 in the emulsion compositions was set to 0.001 to 40% by mass (In other words, the final concentrations of the α-glucosyl naringin composition in test emulsion compositions 1 to 12 were set to 0.001, 0.005, 0.01, 0.1, 0.5, 1, 3, 4, 5, 10, 20, 40% by mass, respectively.), and that sorbitan monostearate (Product Name: RHEODOL AS-10V, Kao Corporation, Japan), which is a non-ionic surfactant, was used as a surfactant, and further that the final concentration of the surfactant in the emulsion composition was set to 0.5% by mass. In addition, two control samples were prepared. Control 1 was prepared using ultrapure water instead of the solution containing the α-glucosyl naringin composition. Control 2 was prepared using ultrapure water instead of the surfactant and prepared such that the final concentration of the α-glucosyl naringin composition was 0.5% by mass.

The prepared test emulsion compositions were incubated for 30 mins at room temperature, moved to a temperature of 30°C, and further incubated at the temperature for 4.5 hours until evaluation. The stability was visually evaluated based on the emulsion state. As an index of the emulsion state of the emulsion composition, the volume of the aqueous layer separated over time was measured by reading scales on a polypropylene container. A relative separation rate was obtained as the volume of the separated aqueous layer measured for test emulsion compositions expressed in a percentage relative to the volume of the separated aqueous layer measured for control 1, regarding the volume of the separated aqueous layer measured for control 1 as 100%. The emulsion stability was judged "⊚" when the relative separation rate was less than 60%, "O" when the relative separation rate was 60% or more and less than 80%, "△" when the relative separation rate was 80% or more and less than 100%, and "X" when the relative separation rate was 100%. The obtained result was shown in Table 4.

**[Table 4]**

| Test Emulsion Composition No. | Control 1 | Control 2 | 1 | 2 | 3 | 4 | 5 | 6 | 7 | 8 | 9 | 10 | 11 | 12 |
|---|---|---|---|---|---|---|---|---|---|---|---|---|---|---|
| α-Glucosyl nanngin composition obtained in Experiment 1-1 (% by mass ) | 0 | 0.5 | 0.001 | 0.005 | 0.01 | 0.1 | 0.5 | 1 | 3 | 4 | 5 | 10 | 20 | 40 |
| Sorbitan monostearate (% by mass) | 0.5 | 0 | 0.5 | 0.5 | 0.5 | 0.5 | 0.5 | 0.5 | 0.5 | 0.5 | 0.5 | 0.5 | 0.5 | 0.5 |
| Sorbitan monostearate: α-Glucosyl naringin composition (% by mass) | - | - | 1:0.002 | 1:0.01 | 1:0.02 | 1:02 | 1:1 | 1:2 | 1:6 | 1:8 | 1:10 | 1:20 | 1:40 | 1:80 |
| Relative Separation Rate (%) | 100 | 100 | 72 | 76 | 68 | 73 | 67 | 57 | 50 | 50 | 53 | 63 | 68 | 85 |
| Evaluation | × | × | ○ | ○ | ○ | ○ | ○ | ⊚ | ⊚ | ⊚ | ⊚ | ○ | ○ | Δ |

As shown in Table 4, the stability test of the emulsion compositions with sorbitan monostearate as a non-ionic surfactant and different final concentrations of the α-glucosyl naringin composition from 0 to 40% by mass revealed that the stability of the emulsion composition was high (judged "○" or "⊚" in the stability test) when the final concentration of the α-glucosyl naringin composition in the emulsion composition was in the range of 0.001 to 20% by mass, or, in other words, when the mass ratio of the glycosyl naringenin (i.e., the α-glucosyl naringin composition) relative to the non-ionic surfactant in the emulsion composition was in the range of 1:0.002 to 1:40. Furthermore, the stability of the emulsion composition was especially high (judged "⊚" in the stability test) when the final concentration of the α-glucosyl naringin composition in the emulsion composition was in the range of 1 to 5% by mass, or, in other words, when the mass ratio of the glycosyl naringenin (i.e., the α-glucosyl naringin composition) relative to the non-ionic surfactant in the emulsion composition was in the range of 1:2 to 1:10.

This result indicates that the remarkably high stabilization effect of the emulsion composition emulsified with a non-ionic surfactant is achieved when the α-glucosyl naringin composition, in other words the glycosyl naringenin, was used at a ratio of from 0.002 to 40 parts by mass, and preferably from 2 to 10 parts by mass, relative to 1 part by mass of the non-ionic surfactant.

### <Experiment 2-2: Effect of Mass Ratio of Glycosyl Naringenin and Surfactants on Stability of Emulsion Compositions - part 2>

Emulsion compositions with different mass ratio of the α-glucosyl naringin composition and a non-ionic surfactant were prepared using the α-glucosyl naringin composition prepared in Experiment 1-1 as the glycosyl naringenin and glyceryl oleate or glyceryl isostearate as the non-ionic surfactant, and the emulsion stability of the emulsion compositions was evaluated.

### <Experiment 2-2-1: Preparation of Test Emulsion Compositions and Emulsion Stability Test>

Test emulsion compositions 13 to 20 comprising glyceryl oleate as a non-ionic surfactant and test emulsion compositions 21 to 28 comprising glyceryl isostearate as a non-ionic surfactant were prepared in accordance with the method described in Experiment 2-1 except that the final concentration of the α-glucosyl naringin composition prepared in Experiment 1-1 in the emulsion compositions was set to 0.001 to 20% by mass (In other words, the final concentrations of the α-glucosyl naringin composition in test emulsion compositions 13 to 20 were set to 0.001, 0.01, 0.1, 0.5, 1, 5, 10, 20% by mass, and similarly the final concentrations of the α-glucosyl naringin composition in test emulsion compositions 21 to 28 were set to 0.001, 0.01, 0.1, 0.5, 1, 5, 10, 20% by mass, respectively.), and that glyceryl oleate (Product Name: NIKKOL MGO) and glyceryl isostearate (Product Name: NIKKOL MGIS), both of which were a non-ionic surfactant, were used as a surfactant in preparing test emulsion composition 13 to 20 and 21 to 28, respectively. Two control samples were prepared. Control 1 was prepared using ultrapure water instead of the solution containing the α-glucosyl naringin composition. Control 2 was prepared using ultrapure water instead of the surfactant and prepared such that the final concentration of the α-glucosyl naringin composition was 0.5% by mass.

The prepared test emulsion compositions were incubated for 30 mins at room temperature, moved to a temperature of 30°C, and further incubated at the temperature for 3 days. The stability was visually evaluated based on the emulsion state. As an index of the emulsion state of the emulsion composition, the volume of the oil layer separated over time was measured by reading scales on a polypropylene container. A relative separation rate was obtained as the volume of the separated oil layer measured for test emulsion compositions expressed in a percentage relative to the volume of the separated oil layer measured for control 1, regarding the volume of the separated oil layer measured for control 1 as 100%. The emulsion stability was judged "⊚" when the relative separation rate was less than 60%, "○" when the relative separation rate was 60% or more and less than 80%, "△" when the relative separation rate was 80% or more and less than 100%, and "X" when the relative separation rate was 100%. The obtained result was shown in Tables 5 and 6.

**[Table 5]**

| Test Emulsion Composition No. | Control 1 | Control 2 | 13 | 14 | 15 | 16 | 17 | 18 | 19 | 20 |
|---|---|---|---|---|---|---|---|---|---|---|
| α-Glucosyl naringin composition obtained in Experiment 1-1 (% by mass) | 0 | 0.5 | 0.001 | 0.01 | 0.1 | 0.5 | 1 | 5 | 10 | 20 |
| Glyceryl oleate (% by mass) | 0.5 | 0 | 0.5 | 0.5 | 0.5 | 0.5 | 0.5 | 0.5 | 0.5 | 0.5 |
| Glyceryl oleate: α-Glucosyl naringin composition (mass ratio) | - | - | 1:0.002 | 1:0.02 | 1:0.2 | 1:1 | 1:2 | 1:10 | 1:20 | 1:40 |
| Relative Separation Rate (%) | 100 | 100 | 66.7 | 65.7 | 31.9 | 0 | 0 | 33.3 | 33.3 | 33.3 |
| Evaluation | × | × | ○ | ○ | ⊚ | ⊚ | ⊚ | ○ | ⊚ | ⊚ |

**[Table 6]**

| Test Emulsion Composition No. | Control 1 | Control 2 | 21 | 22 | 23 | 24 | 25 | 26 | 27 | 28 |
|---|---|---|---|---|---|---|---|---|---|---|
| α-Glucosyl naringin composition obtained in Experiment 1-1 (% by mass) | 0 | 0.5 | 0.001 | 0.01 | 0.1 | 0.5 | 1 | 5 | 10 | 20 |
| Glyceryl isostearate (% by mass) | 0.5 | 0 | 0.5 | 0.5 | 0.5 | 0.5 | 0.5 | 0.5 | 0.5 | 0.5 |
| Glyceryl isostearate: α-Glucosyl naringin composition (mass ratio) | - | - | 1:0.002 | 1:0.02 | 1:0.2 | 1:1 | 1:2 | 1:10 | 1:20 | 1:40 |
| Relative Separation Rate (%) | 100 | 100 | 66.7 | 33.3 | 0 | 33.3 | 0 | 0 | 33.3 | 33.3 |
| Evaluation | × | × | ○ | ⊚ | ⊚ | ⊚ | ⊚ | ⊚ | ⊚ | ⊚ |

As shown in Table 5, the stability test of the emulsion compositions with glyceryl oleate, which is a non-ionic surfactant, as a surfactant and different final concentrations of the α-glucosyl naringin composition from 0 to 20% by mass revealed that the stability of the emulsion composition was high (judged "○" or "⊚" in the stability test) when the final concentration of the α-glucosyl naringin composition, in other words the glycosyl naringenin, in the emulsion composition was in the range of 0.001 to 20% by mass, or, in other words, when the mass ratio of the glycosyl naringenin relative to the surfactant in the emulsion composition was in the range of 1:0.002 to 1:40. Furthermore, the stability of the emulsion composition was especially high (judged "⊚" in the stability test) when the final concentration of the glycosyl naringin in the emulsion composition was in the range of 0.1 to 20% by mass, or, to put it another way, when the mass ratio of the glycosyl naringenin relative to the surfactant in the emulsion composition was in the range of 1:0.2 to 1:40.

Furthermore, as shown in Table 6, the stability test of the emulsion compositions with glyceryl isostearate, which is a non-ionic surfactant, as a surfactant and different final concentrations of the α-glucosyl naringin composition from 0 to 20% by mass revealed that the stability of the emulsion composition was high (judged "○" or "⊚" in the stability test) when the final concentration of the α-glucosyl naringin composition, in other words the glycosyl naringenin, in the emulsion composition was in the range of 0.001 to 20% by mass, or, to put it another way, when the mass ratio of the glycosyl naringenin relative to the surfactant in the emulsion composition was in the range of 1:0.002 to 1:40. Furthermore, the stability of the emulsion composition was especially high (judged "⊚" in the stability test) when the final concentration of the glycosyl naringin in the emulsion composition was in the range of 0.01 to 20% by mass, or, in other words, when the mass ratio of the glycosyl naringenin relative to the surfactant in the emulsion composition was in the range of 1:0.02 to 1:40.

These results are consistent with the above-described result of Experiment 2-1 and indicate that the stabilization effect of the emulsion composition emulsified with a non-ionic surfactant is achieved when the α-glucosyl naringin composition is used at a ratio of from 0.002 to 40 parts by mass relative to 1 part by mass of the non-ionic surfactant.

### <Experiment 3: Effect of Composition of Glycosyl Naringenin on the Stability of Emulsion Compositions>

Emulsion compositions were prepared using glycosyl naringenins comprising 3"-α-glucosyl naringin and naringin at a variety of mass ratios, and the stability of the obtained emulsion compositions was evaluated.

### <Experiment 3-1: Preparation of a Pure 3"-α-Glucosyl Naringin Sample>

The α-glucosyl naringin composition obtained in Experiment 1-1 was dissolved in water/acetonitrile/acetic acid mixture (80/20/0.01(v/v)). The resultant solution was fed to a C18 column for separation of 3"-α-glucosyl naringin and naringin, and a fraction containing 3"-α-glucosyl naringin was collected. Herein, the above-described condition for HPLC analysis was used except that a differential refractometer was used as a detector. The obtained fraction was concentrated *in vacuo* and pulverized, to obtain a pure 3"-α-glucosyl naringin sample in a powder form. The HPLC analysis revealed that the 3"-α-glucosyl naringin purity of the obtained pure 3"-α-glucosyl naringin sample was 99.9% by mass.

### <Experiment 3-2: Preparation of Test Emulsion Composition and Emulsion Stability Test>

Nine kinds of glycosyl naringenin mixtures comprising, as the glycosyl naringenin, 3"-α-glucosyl naringin and naringin at different ratios were prepared by mixing the pure 3"-α-glucosyl naringin sample obtained in Experiment 3-1 (purity: 99.9% by mass) and naringin (purity: 99.4% by mass; Acros Organics B.V.B.A., United States) at ratios shown in Table 7. Test emulsion compositions 29 to 37 were prepared in accordance with the method described in Experiment 2-1 except that the nine kinds of glycosyl naringenin mixtures were used as the glycosyl naringenin, and that the final concentration of the glycosyl naringenin mixture in the emulsion composition was set to 0.5% by mass. As a control, control 1 was prepared using ultrapure water instead of the glycosyl naringenin mixture.

The prepared test emulsion compositions were incubated for 30 mins at room temperature, moved to a temperature of 25°C, and further incubated at the temperature for 4.5 hours until evaluation. The stability was visually evaluated based on the emulsion state. As an index of the emulsion state of the emulsion composition, the volume of the aqueous layer separated over time was measured by reading scales on a polypropylene container. A relative separation rate was obtained as the volume of the separated aqueous layer measured for test emulsion compositions expressed in a percentage relative to the volume of the separated aqueous layer measured for the control composition, regarding the volume of the separated aqueous layer measured for the control composition as 100%. The emulsion stability was judged "⊚" when the relative separation rate was less than 60%, "○" when the relative separation rate was 60% or more and less than 80%, "△" when the relative separation rate was 80% or more and less than 100%, and "X" when the relative separation rate was 100%. The obtained result was shown in Table 7.

**[Table 7]**

| Test Emulsion Composition No. | Control 1 | 29 | 30 | 31 | 32 | 33 | 34 | 35 | 36 | 37 |
|---|---|---|---|---|---|---|---|---|---|---|
| Mass ratio of 3"-α-Glucosyl naringin sample obtained in Experiment 3-1 to naringin | - | 100:0 | 90:10 | 75:25 | 50:50 | 33:67 | 20:80 | 15:85 | 9:91 | 0:100 |
| Relative Separation Rate (%) | 100 | 60 | 53 | 50 | 57 | 33 | 33 | 60 | 57 | 86 |
| Evaluation | × | ○ | ⊚ | ⊚ | ⊚ | ⊚ | ⊚ | ○ | ⊚* | Δ* |

| | | | | | | | | | | |
|---|---|---|---|---|---|---|---|---|---|---|
| * Precipitaion of insoluble matters was observed after evaluation. | | | | | | | | | | |

The stability test was performed using the emulsion compositions with sorbitan monostearate, which is a non-ionic surfactant, as a surfactant and the glycosyl naringenin mixtures comprising 3"-α-glucosyl naringin and naringin at different mass ratios from 100:0 to 0:100. As shown in Table 7, the more or less improved emulsion stability compared to control 1 was observed for all mass ratios of 3"-α-glucosyl naringin and naringin in the range of 100:0 to 0:100. In particular, when the mass ratio of 3"-α-glucosyl naringin and naringin was in the range of 100:0 to 9:91, the relative separation ratio was judged "O" or "⊚", indicating the remarkable improvement of the emulsion stability.

Although the improved emulsion stability was observed also for the emulsion compositions with the mass ratio of 3"-α-glucosyl naringin and naringin of 9:91 and 0:100 in the stability test, precipitation of insoluble matters was observed in these compositions after the test, suggesting, to the extent tested in this experiment, a little bit inferior quality of these emulsion composition as an emulsion composition product. In contrast, no precipitation of insoluble matters was observed after the test in the emulsion composition with the mass ratio of 3"-α-glucosyl naringin and naringin of 15:85, indicating that this emulsion composition had the improved emulsion stability as well as the satisfactory high quality as an emulsion composition product. The mass ratios of 3"-α-glucosyl naringin and naringin of 9:91, 15:85, and 100:0 are equivalent to 1:10.1, 1:5.7, and 1:0, respectively, when the mass of 3"-α-glucosyl naringin is regarded as 1. Therefore, the mass ratio of 3"-α-glucosyl naringin and naringin in the range of 1:0 to 1:10, preferably in the range of 1:0 to 1:8, and more preferably in the range of 1:0 to 1:6 was judged to be preferable from the viewpoint of improving the emulsion stability while avoiding precipitation of insoluble matters.

Furthermore, as shown in Table 7, when the mass ratio of 3"-α-glucosyl naringin and naringin was in the range of 90:10 to 20:80, the relative separation degree was always less than 60% (judged "⊚"), indicating the more remarkable improvement of the emulsion stability compared to when 3"-α-glucosyl naringin was used alone (the mass ratio of 100:0) and to when naringin was used alone (the mass ratio of 0:100). This result indicates that the effect of improving the stability of an emulsion composition shown by the glycosyl naringenin can be synergistically improved and remarkably enhanced when more than 2 kinds of the glycosyl naringenin were used in combination compared to when one kind of the glycosyl naringenin was used alone. The mass ratios of 3"-α-glucosyl naringin and naringin of 90:10 and 20:80 are equivalent to 1:0.1 and 1:4, respectively, when the mass of 3"-α-glucosyl naringin is regarded as 1. Therefore, it is concluded that, when 3"-α-glucosyl naringin and naringin are used in combination, the mass ratio of 3"-α-glucosyl naringin and naringin in the range of 1:0.1 to 1:4 may be preferable and at the mass ratio in this range the remarkably high emulsion stability may be shown.

### <Experiment 3-3: Preparation of Test Emulsion Compositions and Emulsion Stability Test - part 2>

Test emulsion compositions 38 to 44 comprising glyceryl stearate as a non-ionic surfactant and test emulsion compositions 45 to 51 comprising glyceryl isostearate as a non-ionic surfactant were prepared in accordance with the method described in Experiment 3-2 except that the mixtures of the pure 3"-α-glucosyl naringin sample (purity: 99.9% by mass) obtained in Experiment 3-1 and naringin (purity: 99.4% by mass) used in Experiment 3-2 at the mass ratios of 100:0, 90:10, 75:25, 50:50, 33:67, 20:80, 15:85 were used as the glycosyl naringenin, and that glyceryl oleate (Product Name: "NIKKOL MGO") or glyceryl isostearate (Product Name: "NIKKOL MGIS"), which are non-ionic surfactants, was used as a surfactant instead of sorbitan monostearate. Herein, the final concentration of the glycosyl naringenin mixture in the test emulsion compositions were fixed at 0.5% by mass. As a control, control 1 was prepared using ultrapure water instead of the glycosyl naringenin.

The prepared test emulsion compositions were incubated for 30 mins at room temperature, moved to a temperature of 30°C, and further incubated at the temperature for 7 days. The stability was visually evaluated based on the emulsion state. As an index of the emulsion state of the emulsion composition, the volume of the oil layer separated over time was measured by reading scales on a polypropylene container. A relative separation rate was obtained as the volume of the separated oil layer measured for test emulsion compositions expressed in a percentage relative to the volume of the separated oil layer measured for the control composition, regarding the volume of the separated oil layer measured for the control composition as 100%. The emulsion stability was judged "⊚" when the relative separation rate was less than 60%, "○" when the relative separation rate was 60% or more and less than 80%, "△" when the relative separation rate was 80% or more and less than 100%, and "X" when the relative separation rate was 100%. The obtained results were shown in Tables 8 and 9.

**[Table 8]**

| Test Emulsion Composition No. | Control 1 | 38 | 39 | 40 | 41 | 42 | 43 | 44 |
|---|---|---|---|---|---|---|---|---|
| Mass ratio of 3"-α-Glucosyl naringin sample obtained in Experiment 3-1 to naringin | - | 100:0 | 90:10 | 75:25 | 50:50 | 33:67 | 20:80 | 15:85 |
| Relative Separation Rate (%) | 100 | 12.5 | 12.5 | 12.5 | 12.5 | 12.5 | 12.5 | 12.5 |
| Evaluation | × | ⊚ | ○ | ⊚ | ⊚ | ⊚ | ○ | ⊚ |

**[Table 9]**

| Test Emulsion Composition No. | Control 1 | 45 | 46 | 47 | 48 | 49 | 50 | 51 |
|---|---|---|---|---|---|---|---|---|
| Mass ratio of 3"-α-Glucosyl naringin sample obtained in Experiment 3-1 to naringin | - | 100:0 | 90:10 | 75:25 | 50:50 | 33:67 | 20:80 | 15:85 |
| Relative Separation Rate (%) | 100 | 10.0 | 10.0 | 10.0 | 10.0 | 10.0 | 10.0 | 10.0 |
| Evaluation | × | ⊚ | ○ | ⊚ | ⊚ | ⊚ | ⊚ | ⊚ |

The stability test was performed using the emulsion compositions with glyceryl oleate or glyceryl isostearate, which are a non-ionic surfactant, as a surfactant and the glycosyl naringenin mixtures comprising 3"-α-glucosyl naringin and naringin at different ratios from 100:0 to 15:85. As shown in Tables 8 and 9, the remarkably high emulsion stability (judged "⊚") was observed for all the mass ratios of 3"-α-glucosyl naringin and naringin tested in this experiment (i.e., the mass ratio of 3"-α-glucosyl naringin and naringin in the range of 100:0 to 15:85). Furthermore, no precipitation of insoluble matters was observed after the test. These results indicate that the stabilizer of the present invention functions in the emulsion composition emulsified with a glycerin fatty acid type surfactant as a non-ionic surfactant, similarly as in the emulsion composition emulsified with a sorbitan fatty acid type surfactant. These results further indicate that the use of the stabilizer of the present invention in the emulsion composition emulsified with a glycerin fatty acid type surfactant may result in the remarkably high emulsion stability at least when the mass ratio of 3"-α-glucosyl naringin and naringin is in the range of 100:0 to 15:85, or, in other words, 1:0 to 1:6.

The present invention is explained in further detail below with reference to examples. However, the scope of the invention should not be limited to these examples.

### Example 1

### <Stabilizer for an emulsion composition>

Glycosyl naringenin was prepared in accordance with the method described in Example 1 of Japanese Patent Application Publication No. 2002-199896. In short, 50 parts by mass of naringin and 200 parts by mass of dextrin (DE 8) were dissolved in 500 parts by mass of water under heating, and the pH of the resulting solution was adjusted to 7.0 with addition of 2N sodium hydroxide solution. After the adjustment of pH, cyclodextrin glucanotransferase (Hayashibara Co., Ltd., Japan from *Bacillus stearothermophilus* in an amount of 15 units/g-dextrin was added to the solution and the solution was subjected to a reaction for 48 hours at 68°C. After the reaction, the solution was heated to inactivate the enzyme and filtered to obtain a solution comprising α-glycosyl naringin. This solution comprising α-glycosyl naringin was fed to a column packed with a porous synthetic adsorbent (Product Name: DIAION HP-10, Mitsubishi Kasei Kogyo Kabushiki Kaisha, Japan) at a space velocity (SV) of 2. After washing the column with water, the column was fed with 50 vol % ethanol and an eluent was collected. The eluent was concentrated, the remaining solvent was removed, and then the resulting solution was pulverized to obtain an α-glycosyl naringin sample. The HPLC analysis revealed that the α-glycosyl naringin sample was composed of 74% of glucosyl naringin (13% of 3"-α-glucosyl naringin, and in total 61% of 3"-α-,4'-α-diglucosyl naringin and 4'-α-glucosyl naringin) and 26% of naringin in the molar ratio (equivalent to the molar ratio of naringenin aglycone). This sample may be preferably used as the stabilizer for an emulsion composition.

### Example 2

The α-glycosyl naringin sample obtained in Example 1 of the present description was dissolved in water to obtain a solution containing 1% by mass of the α-glycosyl naringin sample. The pH of the obtained solution was adjusted to 4.5. After the adjustment of pH, the solution was added with glucoamylase (Product Name: Glucozyme; Amano Enzyme Inc., Japan) in an amount of 100 units per gram of the α-glycosyl naringin sample, and was then subjected to a reaction for 24 hours at 55°C. The resultant solution was filtered and fed to a column packed with a porous synthetic adsorbent (Product Name: DIAION HP-10, Mitsubishi Kasei Kogyo Kabushiki Kaisha, Japan) at a space velocity (SV) of 2. α-Glycosyl naringin and unreacted naringin in the resultant solution were adsorbed to the adsorbent, but glucose, salts, etc. were eluted without being adsorbed to the adsorbent. The column was then washed with water, and subsequently was fed with an aqueous ethanol solution. The concentration of ethanol in the aqueous ethanol solution was increased in a stepwise manner to collect a fraction comprising α-glycosyl naringin. The collected fraction was concentrated *in vacuo* and pulverized, to obtain an α-glycosyl naringin sample. The HPLC analysis revealed that the α-glycosyl naringin sample was composed of 72% of glucosyl naringin (62% of 3"-α-glucosyl naringin and 7% of 3"-α-,4'-α-diglucosyl naringin and 3% of 4'-α-glucosyl naringin) and 28% of naringin in the molar ratio (equivalent to the molar ratio of naringenin aglycone). This sample may be preferably used as the stabilizer for an emulsion composition.

### Example 3

### <Milky lotion>

In accordance with a conventional method, 0.5 parts by mass of polyoxyethylene behenyl ether, 1 part by mass of polyoxyethylene sorbitol tetraoleate, 1 part by mass of lipophilic glycerin monostearate, 0.5 parts by mass of pyruvate, 0.5 parts by mass of behenyl alcohol, 1 part by mass of avocado oil, 1 part by mass of the α-glucosyl naringin composition obtained in Experiment 1-1, and an appropriate amount of vitamin E and a preservative were dissolved under heating. To the obtained solution, 1 part by mass of sodium L-lactate, 5 parts by mass of 1,3-butylene glycol, 0.1 parts by mass of carboxy vinyl polymer, and 85.3 parts by mass of refined water were added, and then the mixture was emulsified using a homogenizer. After addition of an appropriate amount of a fragrance, the mixture was further homogenized to obtain a milky lotion. This milky lotion could be advantageously used as a sunscreen, skin beautifying agent, skin whitening agent, etc. and showed remarkably high stability as an emulsion composition as well as remarkably good feeling of use. Furthermore, this milky lotion may be used for the purpose of keeping firm and dullness-free skin, since the glycosyl naringenins such as α-glucosyl naringin comprised in the milky lotion has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 4

### <Lotion>

Two (2) parts by mass of stearic acid, 1.5 parts by mass of cetyl alcohol, 4 parts by mass of petrolatum, 5 parts by mass of squalane, 2 parts by mass of glycerin tri(2-ethylhexanoate), 2 parts by mass of polyoxyethylene behenyl ether as a non-ionic surfactant, 5 parts by mass of dipropylene glycol, 3 parts by mass of polyethylene glycol 1500, 1 part by mass of triethanolamine, 1 part by mass of the pure 3"-α-glucosyl naringin sample obtained in Experiment 3-1, 73.5 parts by mass of refined water, and an appropriate amount of a preservative and a fragrance were mixed and emulsified in accordance with a conventional method, to obtain a lotion. This lotion could be advantageously used as a sunscreen, skin beautifying agent, skin whitening agent, etc., and showed remarkably high stability as an emulsion composition as well as remarkably good feeling of use. Furthermore, this lotion may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 5

### <Lotion>

One part by mass of microcrystalline wax, 2 parts by mass of beeswax, 2 parts by mass of lanolin, 20 parts by mass of liquid paraffin, 10 parts by mass of squalene, 7 parts by mass of propylene glycol, 5 parts by mass of palm kernel fatty acid diethanolamide as a non-ionic surfactant, 5 parts by mass of the stabilizer in accordance with an embodiment of the present invention obtained in Example 1, 68.5 parts by mass of refined water, and an appropriate amount of a preservative and a fragrance were mixed and emulsified in accordance with a conventional method, to obtain a lotion. This lotion could be advantageously used as a sunscreen, skin beautifying agent, skin whitening agent, etc., and showed remarkably high stability as an emulsion composition as well as remarkably good feeling of use. Furthermore, this lotion may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 6

### <Cream>

Eight (8) parts by mass of stearic acid, 4 parts by mass of stearyl alcohol, 6 parts by mass of butyl stearate, 5 parts by mass of propylene glycol, 2 parts by mass of glycerin monostearate as a non-ionic surfactant, 0.4 parts by mass of potassium hydroxide, 5 parts by mass of the stabilizer in accordance with an embodiment of the present invention obtained in Example 2, 69.6 parts by mass of refined water, and an appropriate amount of a preservative and a fragrance were mixed and emulsified in accordance with a conventional method, to obtain a cream. This cream could be advantageously used as a sunscreen, skin beautifying agent, skin whitening agent, etc., and showed remarkably high stability as an emulsion composition as well as remarkably good feeling of use. Furthermore, this cream may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 7

### <Cream>

Six (6) parts by mass of stearyl alcohol, 2 parts by mass of stearic acid, 4 parts by mass of hydrogenated lanolin, 9 parts by mass of squalene, 10 parts by mass of octyl dodecanol, 6 parts by mass of 1,3-buthylene glycol, 4 parts by mass of polyethylene glycol 1500, 5 parts by mass of polyoxyethylene behenyl ether as a non-ionic surfactant, 5 parts by mass of the α-glucosyl naringin composition obtained in Experiment 1-1, 49 parts by mass of refined water, and an appropriate amount of a preservative and a fragrance were mixed and emulsified in accordance with a conventional method, to obtain a cream. This cream could be advantageously used as a sunscreen, skin beautifying agent, skin whitening agent, etc., and showed remarkably high stability as an emulsion composition as well as remarkably good feeling of use. Furthermore, this cream may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 8

### <Cream>

Five (5) parts by mass of cetyl alcohol, 3 parts by mass of stearic acid, 5 parts by mass of petrolatum, 10 parts by mass of squalane, 7 parts by mass of glycerin tri(2-ethylhexanoate), 5 parts by mass of dipropylene glycol, 5 parts by mass of glycerin, 2 parts by mass of propylene glycol monostearate as a non-ionic surfactant, 2 parts by mass of polyoxyethylene (20) cetyl alcohol ether, 2 parts by mass of palm kernel fatty acid diethanol amide, 1 part by mass of triethanolamine, 5 parts by mass of the pure 3"-α-glucosyl naringin sample obtained in Experiment 3-1, 49 parts by mass of refined water, and an appropriate amount of a preservative and a fragrance were mixed and emulsified in accordance with a conventional method, to obtain a cream. This cream could be advantageously used as a sunscreen, skin beautifying agent, skin whitening agent, etc., and showed remarkably high stability as an emulsion composition as well as remarkably good feeling of use. Furthermore, this cream may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 9

### <Cream>

Two (2) parts by mass of polyoxyethylene glycol monostearate, 5 parts by mass of self-emulsifying glyceryl monostearate, 1 part by mass of liquid paraffin, 10 parts by mass of glyceryl tricaprylate, and an appropriate amount of a preservative were dissolved under heating in accordance with a conventional method. To the solution, 2 parts by mass of the stabilizer in accordance with an embodiment of the present invention obtained in Example 1, 2 parts by mass of L-lactic acid, 5 parts by mass of 1,3-butylene glycol, and 66 parts by mass of refined water were added, and then the mixture was emulsified using a homogenizer. After addition of an appropriate amount of a fragrance, the mixture was further homogenized to obtain a cream. This cream could be advantageously used as a sunscreen, skin beautifying agent, skin whitening agent, etc., and showed remarkably high stability as an emulsion composition as well as remarkably good feeling of use. Furthermore, this cream may be used for the purpose of keeping firm and dullness-free skin, since the glycosyl naringenin has an effect of absorbing ultraviolet rays, increasing the blood flow in the skin, preventing the generation of reactive oxygen species and lipid peroxides, and strengthening the capillaries and thereby shows an effect of preventing and/or improving the occurrence of skin wrinkles, expression wrinkles and sagging skin, and further an effect of preventing and/or improving skin aging.

### Example 10

### <Lipstick>

In accordance with a conventional method, 3.5 parts by mass of titanium dioxide, 0.5 parts by mass of Red No. 201, 2 parts by mass of Red No. 202, 0.05 parts by mass of Red No. 223, 8 parts by mass of candelilla wax, 30 parts by mass of castor oil, 20 parts by mass of cetyl 2-ethylhexanoate, 4 parts by mass of ceresin, 2 parts by mass of carnauba wax, 11 parts by mass of lanolin, 40 parts by mass of glyceryl diisostearate, 1 part by mass of polyoxyethylene (25) polyoxypropylene (20) 2-tetradecyl ether, 1 part by mass of glycerin, 2 parts by mass of glycosyl trehalose (Product Name: Tornare^{®}; Hayashibara Co., Ltd., Japan), 1 part by mass of the stabilizer in accordance with an embodiment of the present invention obtained in Example 2, and 4 parts by mass of refined water were mixed under heating, to obtain a lipstick. This lipstick showed remarkably high stability as an emulsion composition as well as remarkably good feeling of use. Moreover, this lipstick may be used for the purpose of keeping firm and dullness-free lip, since the glucosyl naringin has an effect of absorbing ultraviolet rays, increasing the blood flow of and around the lip, preventing the generation of reactive oxygen species and lipid peroxides, and strengthening the capillaries and thereby shows an effect of improving the lip color as well as an effect of preventing and/or improving the occurrence of skin wrinkles, expression wrinkles and sagging skin on and around the lip, and further a long-lasting effect of preventing and/or improving aging of the lip. In addition, glossy and non-sticky lip, long-lasting makeup as well as good feeling of use can be achieved when this lipstick is applied to the lip, since this lipstick comprises a glycosyl trehalose. Moreover, because of the anti-oxidation and anti-inflammation effect of glycosyl trehalose and glycosyl naringenin, neither roughing nor inflammation of the lip was observed when this lipstick was applied to the lip, highlighting the advantageously high safety of this lipstick. Furthermore, neither odor production nor deformation was observed even when it was left under high temperature conditions for a long period of time.

### Industrial applicability

The stabilizer for an emulsion composition in accordance with the present invention shows an effect of improving the emulsion stability of an emulsion composition emulsified with a non-ionic surfactant. An emulsion composition comprising the stabilizer in accordance with the present invention may show a remarkably good feeling of use, and accordingly the stabilizer in accordance with the present invention may be advantageously used in the fields of food, cosmetics, quasi-drugs, pharmaceuticals, industrial products, etc. Furthermore, since the surfactant content of the emulsion composition may be reduced by use of the stabilizer in accordance with the present invention, higher formulation flexibility can be achieved as well as higher safety with a reduced risk of causing skin irritation, etc. The industrial applicability of the present invention is great.

## Claims

1. A stabilizer for an emulsion composition emulsified with a non-ionic surfactant, the stabilizer comprising a glycosyl naringenin as an active ingredient.

2. The stabilizer according to claim 1, wherein the glycosyl naringenin is one or more selected from naringin, 3"-α-glucosyl naringin, 3"-α-,4'-α-diglucosyl naringin, 4'-α-glucosyl naringin, prunin, and narirutin.

3. The stabilizer according to claim 1 or 2, wherein the mass ratio of 3"-α-glucosyl naringin to naringin in the glycosyl naringenin is in the range of 1:0 to 1:10.

4. The stabilizer according to any one of claims 1 to 3, wherein the non-ionic surfactant is an ester type surfactant, an ether type surfactant, an amido type surfactant, an ester-ether type surfactant, a polyoxyethylene type surfactant, or an alkyl-polyglucoside type surfactant.

5. The stabilizer according to claim 4, wherein the ester type surfactant is a glycerin fatty acid ester type surfactant, a sorbitan fatty acid ester type surfactant, or a sucrose fatty acid ester type surfactant.

6. The stabilizer according to claim 4, wherein the ether type surfactant is a polyoxyethylene alkyl ether type surfactant, a polyoxyethylene alkyl phenyl ether type surfactant, or a polyoxyethylene-polyoxypropylene glycol type surfactant.

7. The stabilizer according to any one of claims 1 to 6, which is used such that the emulsion composition contains the glycosyl naringenin in a ratio of from 0.002 to 40 parts by mass relative to 1 part by mass of the non-ionic surfactant used.

8. A method for stabilizing an emulsion composition emulsified with a non-ionic surfactant, the method comprising a step of having the stabilizer according to any one of claims 1 to 7 contained in a starting material of the emulsion composition, an intermediate product of the emulsion composition, and/or the emulsion composition manufactured.

9. The method according to claim 8, wherein the stabilizer is used such that the emulsion composition contains the glycosyl naringenin in a ratio of from 0.002 to 40 parts by mass relative to 1 part by mass of the non-ionic surfactant.

## Patentansprüche

1. Stabilisator für eine mit einem nichtionischen Tensid emulgierte Emulsionszusammensetzung, wobei der Stabilisator ein Glykosylnaringenin als Wirkstoff enthält.

2. Stabilisator nach Anspruch 1, wobei das Glykosylnaringenin eines oder mehrere ausgewählt aus Naringin, 3"-α-Glucosylnaringin, 3"-α-,4'-α-Diglucosylnaringin, 4-α-Glucosylnaringin, Prunin und Narirutin ist.

3. Stabilisator nach Anspruch 1 oder 2, wobei das Massenverhältnis von 3"-α-Glucosylnaringin zu Naringin im Glycosylnaringenin im Bereich von 1:0 bis 1:10 liegt.

4. Stabilisator nach einem der Ansprüche 1 bis 3, wobei das nichtionische Tensid ein Tensid vom Estertyp, ein Tensid vom Ethertyp, ein Tensid vom Amidtyp, ein Tensid vom Ester-Ether-Typ, ein Tensid vom Polyoxyethylentyp oder ein Tensid vom Alkylpolyglucosidtyp ist.

5. Stabilisator nach Anspruch 4, wobei das Tensid vom Estertyp ein Tensid vom Glycerinfettsäureestertyp, ein Tensid vom Sorbitanfettsäureestertyp oder ein Tensid vom Saccharosefettsäureestertyp ist.

6. Stabilisator nach Anspruch 4, wobei das Tensid vom Ethertyp ein Tensid vom Polyoxyethylenalkylethertyp, ein Tensid vom Polyoxyethylenalkylphenylethertyp oder ein Tensid vom Polyoxyethylen-Polyoxypropylenglykoltyp ist.

7. Stabilisator nach einem der Ansprüche 1 bis 6, der so verwendet wird, dass die Emulsionszusammensetzung das Glykosylnaringenin in einem Verhältnis von 0,002 bis 40 Masseteilen, bezogen auf 1 Masseteil des verwendeten nichtionischen Tensids, enthält.

8. Verfahren zum Stabilisieren einer mit einem nichtionischen Tensid emulgierten Emulsionszusammensetzung, wobei das Verfahren einen Schritt beinhaltet, bei dem der Stabilisator nach einem der Ansprüche 1 bis 7 in einem Ausgangsmaterial der Emulsionszusammensetzung, einem Zwischenprodukt der Emulsionszusammensetzung und/oder der hergestellten Emulsionszusammensetzung enthalten ist.

9. Verfahren nach Anspruch 8, wobei der Stabilisator so verwendet wird, dass die Emulsionszusammensetzung das Glycosylnaringenin in einem Verhältnis von 0,002 bis 40 Masseteilen, bezogen auf 1 Masseteil des nichtionischen Tensids, enthält.

## Revendications

1. Un agent stabilisant pour une composition d'émulsion émulsifiée à l'aide d'un agent tensioactif non ionique, l'agent stabilisant comprenant une naringénine glycosylée en tant que principe actif.

2. L'agent stabilisant selon la revendication 1, dans lequel la naringénine glycosylée est un ou plusieurs éléments sélectionnés parmi la naringine, la 3"-α-glucosyl naringine, la 3"-α-,4'-α-diglucosyl naringine, la 4'-α-glucosyl naringine, la prunine et la narirutine.

3. L'agent stabilisant selon la revendication 1 ou 2, dans lequel le rapport en masse de la 3"-α-glucosyl naringine à la naringine dans la naringénine glycosylée est dans la plage de 1:0 à 1:10.

4. L'agent stabilisant selon l'une quelconque des revendications 1 à 3, dans lequel l'agent tensioactif non ionique est un agent tensioactif de type ester, un agent tensioactif de type éther, un agent tensioactif de type amido, un agent tensioactif de type ester-éther, un agent tensioactif de type polyoxyéthylène ou un agent tensioactif de type alkyl- polyglucoside.

5. L'agent stabilisant selon la revendication 4, dans lequel l'agent tensioactif de type ester est un agent tensioactif de type ester d'acide gras de glycérine, un agent tensioactif de type ester d'acide gras de sorbitan ou un agent tensioactif de type ester d'acide gras de sucrose.

6. L'agent stabilisant selon la revendication 4, dans lequel l'agent tensioactif de type éther est un agent tensioactif de type éther d'alkyle de polyoxyéthylène, un agent tensioactif de type éther d'alkyle phényle de polyoxyéthylène ou un agent tensioactif de type polyoxyéthylène- polyoxypropylène glycol.

7. L'agent stabilisant selon l'une quelconque des revendications 1 à 6, lequel est utilisé de telle sorte que la composition d'émulsion contient la naringénine glycosylée dans un rapport de 0,002 à 40 parties en masse pour 1 partie en masse de l'agent tensioactif non ionique utilisé.

8. Un procédé de stabilisation d'une composition d'émulsion émulsifiée à l'aide d'un agent tensioactif non ionique, le procédé comprenant une étape consistant à ce que l'agent stabilisant selon l'une quelconque des revendications 1 à 7 soit contenu dans une matière de départ de la composition d'émulsion, d'un produit intermédiaire de la composition d'émulsion et/ou de la composition d'émulsion fabriquée.

9. Le procédé selon la revendication 8, dans lequel l'agent stabilisant est utilisé de telle sorte que la composition d'émulsion contient la naringénine glycosylée dans un rapport de 0,002 à 40 parties en masse pour 1 partie en masse de l'agent tensioactif non ionique.
